# FASCICULE DE BREVET EUROPEEN

(11) **EP 0 575 478 B1**
(45) Date de publication et mention de la délivrance du brevet: **10.09.1997**
(21) Numéro de dépôt: 92907845.9
(22) Date de dépôt: 12.03.1992
(51) Int. Cl.: A61F 2/02

(54) **FILTRE ANTI-EMBOLIE PULMONAIRE PERFECTIONNE ET KIT DE PRESENTATION ET MISE EN PLACE CORRESPONDANTE**
Lungenemboliefilter sowie Bausatz zum Präsentieren und Einsetzen desselben
IMPROVED PULMONARY EMBOLISM PREVENTION FILTER AND ASSOCIATED POSITIONING AND FITTING KIT

(30) Priorité: 14.03.1991 FR 9103103; 19.11.1991 FR 9114231
(43) Date de publication de la demande: 29.12.1993
(73) Titulaire: ETHNOR, F-92523 Neuilly sur Seine Cédex (FR)
(72) Inventeur: DIBIE, Alain, C.M.C. Porte de Choisy, F-75013 Paris (FR); MUSSET, Dominique, Hôpital Antoine-Béclère, F-92140 Clamart (FR); PROU, Philippe, Ethnor S.A., F-28702 Auneau Cédex (FR); CATTEAU, Gilles, Ethnor S.A., F-28702 Auneau Cédex (FR)
(74) Mandataire: Martin, Jean-Jacques
(86) Numéro de dépôt international: FR9200224
(87) Numéro de publication internationale: WO9216163

(56) Documents cités:
- EP-A- 0 121 447
- EP-A- 0 323 333
- DE-A- 3 203 410
- FR-A- 2 616 666
- FR-A- 2 645 731
- US-A- 4 994 069

## Description

La présente invention concerne le domaine des filtres anti-embolie pulmonaire.

Plus précisément, la présente invention concerne des perfectionnements au filtre anti-embolie pulmonaire décrit dans les demandes de brevet n° 89 13160 du 9 Octobre 1989 et n° 89 16538 du 14 Décembre 1989 et dans la demande de brevet PCT n° PCT/FR90/00721 déposée le 9 Octobre 1990.

Différents filtres anti-embolie pulmonaire ont déjà été proposés. On peut sur ce point se référer par exemple aux documents FR-A-2616666, EP-A-121447, EP-A-323333 et DE-A-3203410.

Les filtres anti-embolie pulmonaire décrits dans les documents précités ne donnent toutefois pas totalement satisfaction.

Le document EP-A-323333 en particulier décrit un filtre en fil métallique élastique à effet de ressort.

Sa forme géométrique particulière détermine deux boucles en forme d'ellipses d'axes perpendiculaires et égaux, coplanaires, donnant quatre points de contacts permanents avec la paroi de la veine cave dans laquelle il est implanté.

Ce dernier type de filtre a effectivement fait l'objet de réalisation et expérimentation sur l'animal, puis sur l'homme. Toutefois, de telles difficultés d'ordre médical sont apparues, tant sur la procédure de mise en place du filtre que sur son mode de fonctionnement une fois en place, que la généralisation d'emploi de ce filtre ne peut être recommandée médicalement.

Plus précisément, une première difficulté réside en ce que le filtre, qui a initialement la forme précitée en double ellipses d'axes perpendiculaires, après sa mise en forme momentanée rectiligne à l'intérieur du cathéter de mise en place, risque parfois, lors de sa libération hors du cathéter, à l'intérieur de la veine cave inférieure, de ne pas reprendre sa forme initiale. Cet inconvénient résulte des parties quasi rectilignes assez longues qui relient les parties quasi circulaires des quasi ellipses allongées. Ainsi, il y a des risques inacceptables de voir le filtre se retrouver en forme de S allongé ou de 8 et non plus en ellipses croisées. L'appui contre les parois de la veine cave en quatre points équilibrés n'est plus obtenu et, de ce fait, la veine n'est pas suffisamment aplatie, de sorte que le filtre peut migrer en se déplaçant le long de la veine après sa mise en place.

Par ailleurs, les plans de ces ellipses sont quasi coplanaires et l'effet de filtre n'est garanti que si les quatre points d'appui du filtre contre la paroi de la veine cave sont en bonne position, ce qui n'est déjà pas garanti comme on l'a exposé ci-dessus d'une part, et aplatissent très fortement la veine cave pour réduire fortement la section droite de la veine et obtenir ainsi l'effet de filtre, d'autre part.

Enfin, une extrémité du filtre est équipé d'une pointe acérée inclinée par rapport au plan des ellipses et servant de point d'ancrage dans la veine cave. Cette pointe acérée présente deux inconvénients : elle rend délicate son introduction dans le cathéter, d'une part, et risque de traumatiser ou même perforer la paroi de la veine, d'autre part.

Le filtre décrit dans les demandes de brevet français n° 89 13160 du 9 Octobre 1989 et n° 89 16538 du 14 Décembre 1989 ainsi que dans la demande de brevet PCT correspondante n° PCT/FR90/00721 du 9 Octobre 1990 a permis d'améliorer la situation. Ce filtre comprend un fil élastique à effet de ressort rémanent conformé en une spirale à trois spires non jointives dont la spire médiane a un diamètre supérieur à ceux des deux autres spires, le diamètre de la spire médiane étant de préférence choisi voisin de la valeur de la demi-circonférence de la veine cave dans la zone où le filtre doit être implanté pour assurer optimalement la fonction de maintien en place du filtre par aplatissement de la veine cave.

Le but de la présente invention est de perfectionner encore le filtre du type précité en améliorant l'effet de filtrage obtenu, tout en autorisant à la fois une mise en place et un retrait aisé du filtre.

Ce but est atteint selon la présente invention grâce à la structure définie dans la revendication 1 annexée.

D'autres caractéristiques, buts et avantages de la présente invention apparaîtront à la lecture de la description détaillée qui va suivre, et en regard des dessins annexés donnés à titre d'exemples non limitatifs et sur lesquels :
- la figure 1 représente une vue schématique en perspective d'un filtre anti-embolie pulmonaire conforme à un mode de réalisation préférentiel de la présente invention,
- la figure 2 représente une vue de dessus du même filtre conforme au mode de réalisation préférentiel de la présente invention,
- la figure 3 représente une vue latérale du même filtre conforme à la présente invention,
- les figures 4, 5 et 6 représentent schématiquement, à l'état tendu semi-rectiligne, les fils formant trois variantes de réalisation du filtre conforme à la présente invention,
- la figure 7 représente une vue schématique en perspective d'un filtre anti-embolie pulmonaire conforme à une variante de réalisation de la présente invention,
- la figure 8 représente une vue schématique en perspective d'un filtre anti-embolie pulmonaire conforme à une autre variante de réalisation de la présente invention,
- la figure 9 représente une vue partielle d'une extrémité du filtre conforme à la présente invention associée à un premier poussoir de mise en place,
- la figure 10 représente une vue similaire à la figure 9, selon une coupe longitudinale du même poussoir de mise en place,
- la figure 11 représente une vue partielle d'une extrémité du filtre conforme à la présente invention associée à un autre poussoir de mise en place,
- la figure 12 représente la même extrémité du filtre et le même poussoir de mise en place que la figure 11, mais à l'état séparé,
- les figures 13 et 14 illustrent les étapes de mise en place du filtre dans une veine cave,
- la figure 15 représente une vue en coupe transversale d'une veine cave équipée d'un filtre conforme à la présente invention,
- la figure 16 représente schématiquement un kit de présentation et mise en place conforme à la présente invention,
- la figure 17 représente une vue schématique d'un dispositif de retrait de type lasso,
- la figure 18 représente un premier mode de réalisation d'une pince intra-cathéter conforme à la présente invention conçue pour le retrait du filtre anti-embolie pulmonaire,
- la figure 19 représente une vue de détail, à échelle agrandie, de la même pince,
- la figure 20 représente une vue partielle de la même pince, à l'état actionné,
- la figure 21 représente une autre vue de détail, à échelle agrandie, de la même pince,
- les figures 22, 23, 24, 25 et 26 représentent différentes étapes du fonctionnement de cette pince,
- la figure 27 représente un second mode de réalisation d'une pince intra-cathéter conforme à la présente invention, et
- la figure 28 représente une vue de détail, à échelle agrandie de ce second mode de réalisation de la pince intra-cathéter conforme à la présente invention.

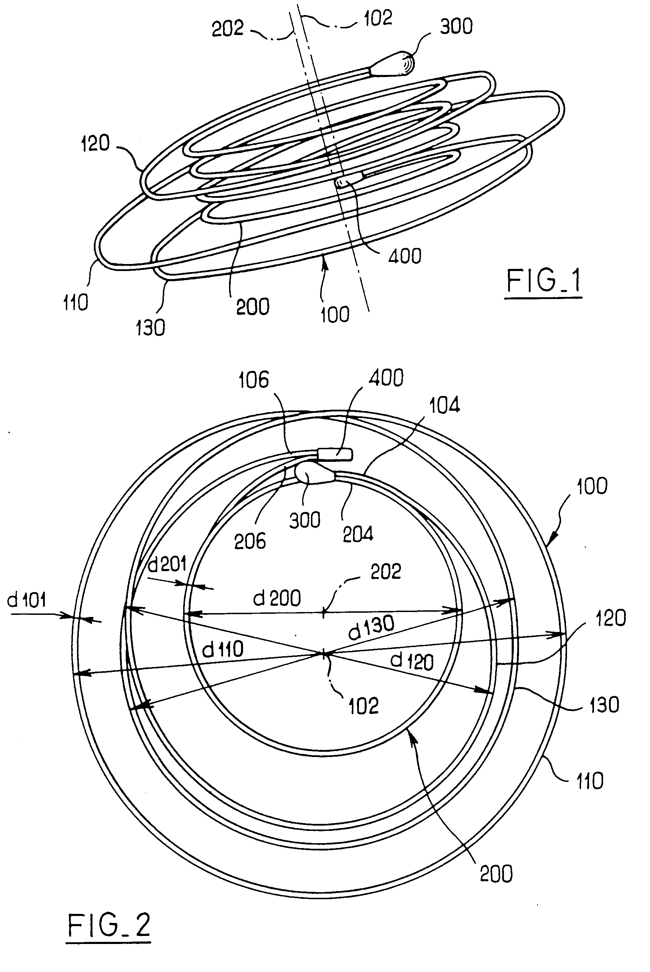

### PREMIER MODE DE REALISATION DU FILTRE REPRESENTE SUR LES FIGURES 1 A 3

On va dans un premier temps décrire le mode de réalisation du filtre anti-embolie pulmonaire représenté sur les figures 1 à 3.

Le filtre représenté sur les figures 1 à 3 est formé de deux fils distincts 100, 200 conformés chacun en spirale non plane.

Plus précisément encore, le fil 100 forme une spirale à trois spires : une spire médiane 110 et deux spires latérales 120, 130. Les trois spires 110, 120, 130 sont non jointives. La spire médiane 110 a un diamètre d110 supérieur aux diamètres moyens d120, d130, des deux spires latérales 120, 130. De préférence les deux spires latérales 120, 130 ont des diamètres moyens d120, d130, identiques ou sensiblement identiques.

Comme on le voit bien à l'examen de la figure 2, les trois spires 110, 120, 130 sont à peu près coaxiales et centrées sur un axe 102 orthogonal au plan de la figure 2. De préférence, le diamètre d110 de la spire médiane 110 est supérieure à 1,25 fois le diamètre d120, d130 des spires latérales 120, 130. Très avantageusement le diamètre d110 de la spire médiane 110 est supérieur à 1,4 fois le diamètre d120, d130 des spires latérales 120, 130.

Les trois spires 110, 120, 130 ont de préférence des pas sensiblement identiques.

Le pas p1 de la spirale formée par le fil 100 est au minimum de l'ordre de 3mm, de préférence au minimum de l'ordre de 7mm, très préférentiellement au minimum de l'ordre de 9mm au repos, avant implantation. Ainsi l'épaisseur axiale totale de la spirale formée par le fil 100 est au minimum d'environ 9mm, de préférence d'environ 21 mm, très préférentiellement d'environ 27mm au repos, quelle que soit la valeur du diamètre d110 de la plus grande spire médiane 110.

La taille du filtre doit être adaptée à la veine cave à appareiller. Pour cela, le diamètre d110 de la spire médiane 110, la plus grande doit se rapprocher le plus possible de la valeur de la demi-circonférence de la veine cave inférieure. Ainsi, la spire médiane 110 a toujours un diamètre d110 plus grand que celui de la veine cave pour obtenir un effet d'aplatissement de la veine cave lors de la mise en place du filtre.

En pratique, on peut choisir une taille 1 de filtre dont le diamètre d110 de la spire médiane 110 est de l'ordre de 27mm pour les veines caves de diamètre 16 à 18 mm. On peut choisir une taille 2 de filtre dont le diamètre d110 de la spire médiane 110 est de l'ordre de 31,5mm pour des diamètres de veines caves de 19 à 21mm. On peut choisir une taille 3 de filtre dont le diamètre d110 de la spire médiane 110 est de l'ordre de 36mm pour des diamètres de veines caves de 22mm à 23mm.

Enfin, on peut choisir une taille 4 de filtre dont le diamètre d110 de la spire médiane 110 est de l'ordre de 40mm pour des veines caves de diamètres supérieurs.

Les extrémités du filtre, c'est-à-dire les extrémités du fil 100 sont radio-opaques aux rayons X pour être bien visibles sous amplificateur de luminance. Cette disposition a pour but de faciliter le contrôle de la mise en place du filtre. Pour cela, les extrémités du fil 100 peuvent être munies d'embout 300, 400 comme cela sera décrit plus en détail par la suite.

Le deuxième fil 200 forme une deuxième spirale. De préférence, la deuxième spirale formée par le fil 200 comprend plusieurs spires de diamètre identique d200. Par ailleurs, la longueur du deuxième fil 200 est au moins sensiblement identique à la longueur du premier fil 100. La seconde spirale formée par le fil 200 a de préférence un pas p2 constant ou sensiblement constant. Le nombre de spires de la deuxième spirale formée par le fil 200 dépend par conséquent du diamètre d200 des spires de la seconde spirale. A titre d'exemple non limitatif, la deuxième spirale formée par le fil 200 possède quatre spires. Selon une caractéristique avantageuse de la présente invention, le diamètre moyen d200 des spires de la seconde spirale formée par le fil 200 est compris entre les deux tiers et la moitié du diamètre d110 de la plus grande spire 110 de la première spirale.

Ainsi par exemple, la seconde spirale formée par le fil 200 a typiquement un diamètre moyen d200 compris entre 14 et 24mm, très avantageusement compris entre 20 et 24mm.

Selon le mode de réalisation représenté sur les figures 1 à 3, les deux spirales formées respectivement par les fils 100 et 200 sont de même sens, c'est-à-dire de même chiralité.

Selon une autre caractéristique avantageuse de la présente invention, le fil 200 composant la seconde spirale de diamètre constant d200 est plus souple que le fil 100 composant la première spirale. Pour cela, le fil 200 possède de préférence un diamètre inférieur à celui du filtre 100. La seconde spirale 200 est centrée sur un axe 202 orthogonal au plan de la figure 2. L'axe 202 est excentré par rapport à l'axe 102 de la premère spirale et parallèle à celui-ci.

Une première extrémité 104 de la première spirale est fixée à une première extrémité 204 de la seconde spirale 200, tandis que la seconde extrémité 106 de la première spirale est fixée à la seconde extrémité 206 de la seconde spirale.

Les deux fils 100, 200 peuvent être reliés, par leurs extrémités respectives, à l'aide de tous moyens classiques appropriés. Les fils 100, 200 peuvent ainsi être reliés par collage ou brasure à leurs extrémités.

Cependant, selon un mode de réalisation préférentiel, les deux fils 100, 200 sont reliés entre eux, à leurs extrémités, par sertissage dans les embouts 300, 400.

Ces embouts 300, 400 peuvent faire l'objet de nombreux modes de réalisation.

Il s'agit de préférence de corps généralement arrondis pour éviter de blesser la veine cave.

Par la suite, on appellera extrémité proximale, l'extrémité du filtre formée par les extrémités 104 et 204 des fils 100, 200. On appellera par ailleurs extrémité distale du filtre, l'extrémité formée par les extrémités 106 et 206 des fils.

Selon le mode de réalisation représenté sur les figures 1 à 3, l'embout 300 a une forme ovoîde en goutte d'eau, arrondi vers son bout libre et conique en son bout serti sur les extrémités 104 et 204 des fils 100, 200. Cette forme lui permet de pouvoir se déplacer dans les deux sens à l'intérieur de la lumière d'un cathéter de mise en place sans endommager celui-ci et sans contrainte. La forme ovoîde en goutte d'eau de l'embout 300 permet également d'éviter toute blessure de la veine cave inférieure. Enfin, la forme ovoîde en goutte d'eau de l'embout 300 permet de réintroduire sans difficulté le filtre à l'intérieur d'un cathéter de retrait, si nécessaire, sans buter sur l'orifice d'entrée du cathéter.

A titre d'exemple non limitatif, les dimensions de l'embout 300 peuvent être de 1,3mm pour son plus grande diamètre et de 0,7mm pour son diamètre de raccordement sur les fils 100, 200, la longueur de l'embout 300 étant de l'ordre de 3,5mm.

Le second embout 400 peut être identique à l'embout 300 précédemment décrit, c'est-à-dire avoir une forme ovoîde en goutte d'eau.

Toutefois, selon le mode de réalisation particulier représenté sur les figures 1 et 2, le second embout 400 a la forme d'un petit cylindre à extrémités arrondies. A titre d'exemple non limitatif, le cylindre 400 a une diamètre externe de l'ordre de 0,8mm et une longueur de l'ordre de 4mm. Il est serti sur les extrémités distales 106, 206 des fils 100 et 200.

Le cas échéant, l'embout 300 premier cité peut être identique à l'embout 400 : c'est-à-dire que l'embout 300 peut être formé d'un petit cylindre à extrémités arrondies.

Les embouts 300, 400 peuvent être pourvus d'un trou unique recevant à la fois les extrémités proximales 104 et 204 des fils 100, 200 ou les extrémités distales 106, 206 de ceux-ci. En variante les embouts 300, 400 peuvent être pourvus chacun de deux trous recevant respectivement les extrémités proximales 104, 204 ou les extrémités distales 106, 206 des fils.

Selon le mode de réalisation représenté sur les figures 1 à 3, les fils 100 et 200 possèdent une courbure sensiblement régulière et continue sur toute leur longueur. Il en résulte qu'une fois le filtre mis en place, les embouts 300 et 400 reposent contre la surface interne de la veine cave inférieure.

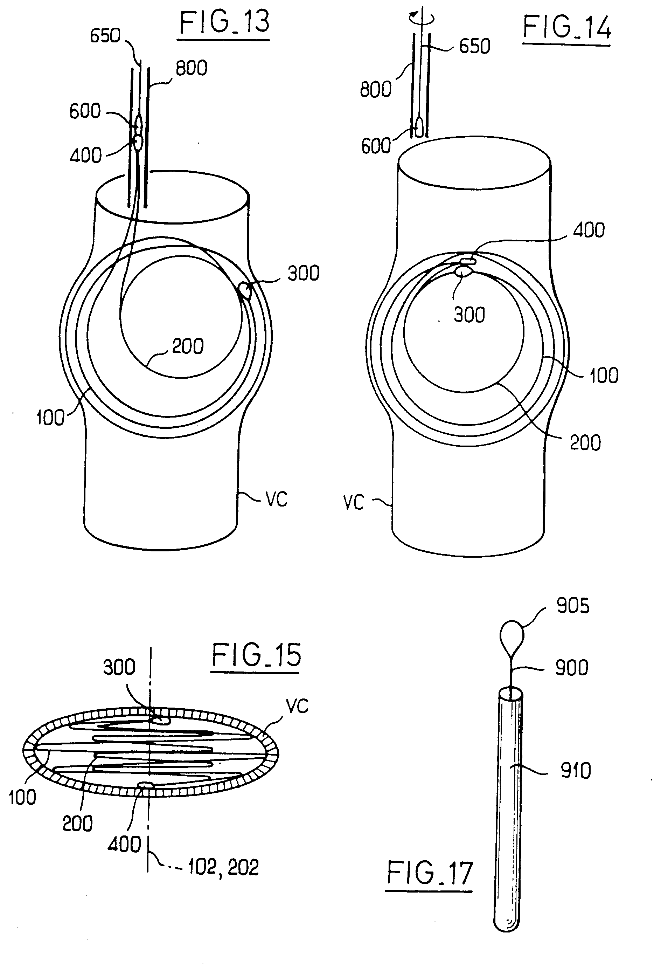

Pour faciliter le retrait du filtre par saisie de l'un des embouts 300, 400, de préférence l'une au moins des extrémités proximales du filtre présente une discontinuité de courbure et converge pour cela à la fois en direction de l'axe 102 de la première spirale et en direction de la spire médiane de plus grand diamètre 110. De ce fait, après implantation du filtre, l'un au moins des embouts 300, 400 correspondant reste éloigné de la surface interne de la veine cave inférieure ce qui facilite la préhension de cette embout, en vue du retrait du filtre, par des moyens tels que décrits dans les demandes de brevet français n° 89 13160 du 9 Octobre 1989, n° 89 16538 du 14 Décembre 1989 ou encore par les moyens représentés sur les figures 17 à 28 et qui seront décrits plus en détail par la suite.

Les fils 100, 200 sont réalisés en matériau élastique, très préférentiellement métallique. Les fils 100, 200 doivent être non magnétisables pour permettre sans dommage au porteur d'un filtre d'être soumis à un examen RMN ou IRM (Imagerie à Résonnance Magnétique). Ce fil doit aussi être radio-opaque pour pouvoir être visible lors d'un examen aux rayons X.

Les fils 100, 200 peuvent être formés de tous matériaux appropriés connus de l'homme de l'art.

A titre d'exemple non limitatif, les fils 100, 200 peuvent être en un alliage choisi dans le groupe comprenant les alliages suivants :
i) cuivre et nickel et aluminium
ii) cuivre et zinc et aluminium
iii) cuivre et zinc et aluminium et nickel
iv) cuivre et étain et nickel.

Un revêtement final d'une mince couche bio-compatible, de préférence d'or, est effectué pour éviter des phénomènes de corrosion et pour assurer la bio-compatibilité.

Le diamètre d101 du premier fil 100 est de préférence compris entre 0,25 et 1mm. Le diamètre d101 du premier fil 100 est typiquement de l'ordre de 0,37mm. Le diamètre d201 du second fil 200 est également de préférence compris entre 0,25mm et 1mm. Comme indiqué précédemment le diamètre d201 du second fil 200 est de préférence inférieur au diamètre d101 du premier fil 100 de sorte que la seconde spirale formée par le fil 200 soit plus souple que la première spirale formée par le fil 100. Le diamètre d201 du second fil 200 est typiquement de l'ordre de 0,30mm.

Ainsi le filtre formé par les deux fils 100, 200 peut être introduit dans un cathéter 800 de type 7F, c'est-à-dire, la grandeur F valant 0,33mm, un cathéter de diamètre externe de 2,31mm et un calibre utile intérieur de l'ordre de 1,77mm.

L'utilisation d'un cathéter 7F pour la mise en place du filtre et le retrait de celui-ci permet la mise en place du filtre et de retrait par le canal de petites veines, par exemple d'une veine du pli du coude.

Les deux fils 100, 200 sont de préférence réalisés dans le même alliage. Toutefois, les deux fils 100, 200 peuvent être réalisés en alliage différent.

Pour réaliser le filtre représenté sur les figurs 1 à 3, on procède essentiellement comme suit. Dans un premier temps, les deux fils 100, 200 sont sectionnés à longueur voulue. La longueur totale des fils est typiquement comprise entre 22 et 35cm. Les deux fils 100, 200 sont enroulés sur des mandrins de calibre approprié. Le mandrin d'enroulage du premier fil 100 présente plusieurs sections correspondant aux diamètres respectifs d110, d120, d130, de la spire médiane 110 et des spires latérales 120, 130. Le mandrin d'enroulage du second fil 200 présente une section constante égale au diamètre d200 des spires de la seconde spirale.

Une fois enroulés sur ce ou ces mandrins, les fils 100, 200 sont soumis à un traitement thermique approprié connu des spécialistes de la technologie de fils métalliques à caractéristique de ressort à effet rémanent.

L'opération de liaison des deux fils 100, 200, typiquement par sertissage des embouts 300, 400, peut ensuite être réalisée.

Enfin, comme indiqué précédemment, le filtre est revêtu d'un matériau bio-compatible, de préférence d'or, y compris sur les embouts 300, 400.

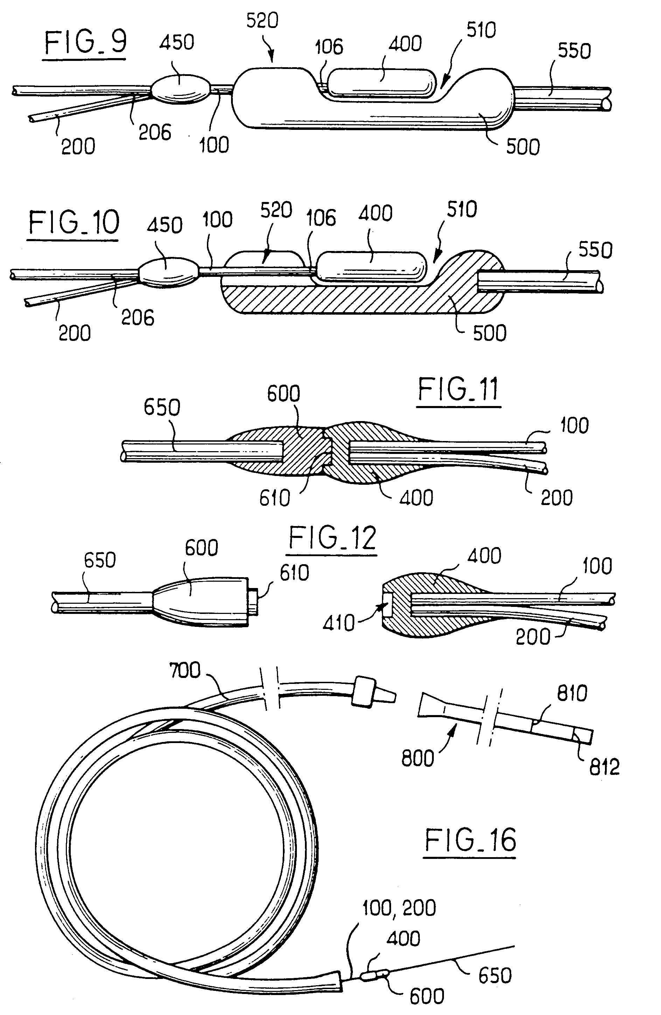

Le mode de mise en place et de retrait éventuel du filtre représenté sur les figures 1 à 3 sera décrit par la suite en regard des figures 9 à 15.

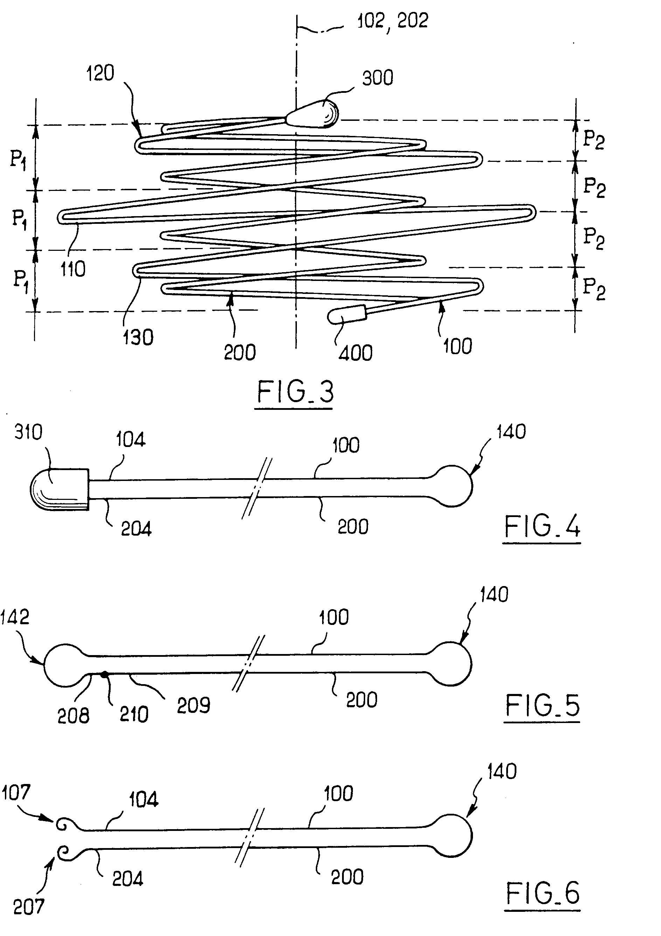

### VARIANTES DE REALISATION REPRESENTEES LES FIGURES 4, 5 ET 6

Selon le mode de réalisation précédemment décrit représenté sur les figures 1 à 3, le filtre est formé de deux fils séparés 100, 200 reliés par leurs extrémités. Les fils 100, 200 peuvent être reliés entre eux par sertissage d'embouts 300, 400 ou par tout autre moyen approprié, tel que par exemple par collage ou brasure.

En variante le filtre anti-embolie pulmonaire conforme à la présente invention peut être réalisé à l'aide d'un fil unique comme représenté sur les figures 4 à 6.

Selon le mode de réalisation représenté sur la figure 4, le fil unique est replié sur lui-même sensiblement à mi-longueur, en 140, pour former les deux spirales 100, 200.

Les extrémités libres 104, 204 des deux brins de fil sont reliées par tout moyen classique approprié. Il peut s'agir d'un collage ou d'une brasure. Selon la figure 4, il s'agit d'une liaison à l'aide d'un embout serti 310. L'embout 310 peut être conforme à l'embout 300 ou à l'embout 400 précédemment décrits. Le coude 140 du fil peut former l'extrémité distale de filtre, auquel cas les extrémités 104, 204 du fil forment l'extrémité proximale du filtre. Inversement le coude 140 du fil peut former l'extrémité proximale du filtre, auquel cas les extrémités 104, 204 du fil forment l'extrémité distale du filtre.

Selon la figure 5, le fil unique est replié deux fois sur lui-même en 140, 142. Les brins 100, 200 du fil formés entre les coudes 140, 142 sont sensiblement de même longueur. Par ailleurs les deux extrémités du fil 208, 209 sont reliées entre elles en 210 de sorte que le fil forme une boucle fermée. La liaison 210 peut être formée par collage, brasure, sertissage d'une olive sur les extrémités 208, 209 ou tout autre moyen approprié.

Selon la figure 6, le fil unique est replié sur lui-même sensiblement à mi-longueur, en 140, pour former les deux spirales 100, 200.

Les extrémités libres 104, 204 des deux brins du fil sont séparées, mais munies chacune d'une boucle ouverte 107, 207, de sorte que le fil ne risque pas de blesser la veine cave recevant le filtre.

Les variantes à fil unique peuvent être utilisées pour réaliser un filtre de même géométrie que celui représenté sur les figures 1 à 3, soit un filtre comprenant une première spirale 100 à trois spires dont une spire médiane de grand diamètre et deux spires latérales de diamètre plus faible, et une deuxième spirale 200 de même sens que la première spirale, comprenant un nombre plus important de spires de diamètre sensiblement constants.

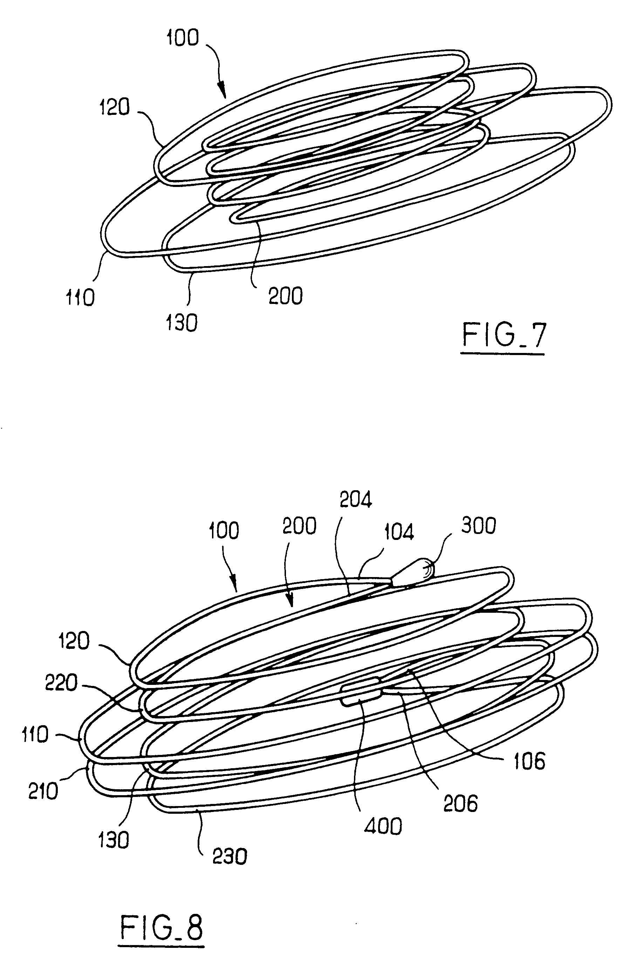

### VARIANTE DE REALISATION REPRESENTEE SUR LA FIGURE 7

Le filtre représenté sur la figure 7 est réalisé à l'aide d'un fil unique conformé en boucle fermée comme illustrée sur la figure 5 précédemment décrite.

Par ailleurs, le filtre représenté sur la figure 7 a la même géométrie que celui représenté sur les figures 1 à 3. Ainsi le filtre représenté sur la figure 7 comprend deux spirales 100, 200.

La première spirale 100 comprend trois spires : une spire médiane 110 de grand diamètre et deux spires latérales 120, 130 de diamètres sensiblement identiques entre eux mais plus faible que la spire médiane 110.

La seconde spirale 200 comprend plus de spires que la première spirale 100, quatre spires par exemple, et ses spires sont de diamètres sensiblement constants.

On notera que si selon les figures 1 à 3 les deux spirales 100, 200 sont de même sens. En revanche selon la figure 7, les deux spires sont de sens opposés. C'est-à-dire que les deux spires 100, 200 présentent des chiralités opposées selon la figure 7.

L'utilisation des deux spirales 100, 200 de même sens permet de faciliter le déploiement du filtre dans la veine cave, à la sortie du cathéter de mise en place, puisque les deux spirales 100, 200 se déploient alors dans la même direction générale.

En revanche, l'utilisation de deux spirales 100, 200 de sens opposés permet de faciliter le déplacement du filtre dans le cathéter de mise en place, puisque les deux spirales prennent alors appui sur des zones diamétralement opposées de ce cathéter et génèrent des efforts sensiblement symétriques, ce qui évite un blocage du filtre dans le cathéter.

En variante, le filtre comprenant deux spirales 100, 200, de sens opposés, représenté sur la figure 7 pourrait être réalisé avec un fil unique du type illustré sur la figure 4 ou sur la figure 6 ou encore avec deux fils distincts reliés à leurs extrémités par collage, brasure, sertissage d'embouts ou tout autre moyen approprié comme indiqué précédemment.

Dans le cas où comme représenté sur la figure 6, les extrémités libres 104, 204 du filtre unique restent séparées, on peut prévoir que les deux spirales 100, 200 aient des longueurs au moins légèrement différentes.

Selon une autre variante dérivée des figures 1 et 6, on peut prévoir un filtre formé à l'aide de deux fils distincts reliés par une seule de leurs extrémités, par collage, brasure, sertissage d'embout, ou tout autre moyen approprié, mais laissés libres à leurs secondes extrémités. Dans ce cas, il est préférable de prévoir alors des boucles similaires aux boucles 107, 207 sur ces secondes extrémités des fils 100, 200 pour éviter de blesser la veine cave. Si les deux fils 100, 200 restent séparés à l'une de leurs extrémités, ils peuvent présenter des longueurs au moins légèrement différentes.

### VARIANTE DE REALISATION REPRESENTEE SUR LA FIGURE 8

Ainsi par exemple, comme représenté sur la figure 8, on peut envisager de donner aux deux spirales 100, 200 des conformations sensiblement identiques entre elles et identiques à la conformation de la première spirale formée par le fil 100 sur les figures 1 et 2.

Dans ce cas, les deux spirales 100, 200 comprennent chacune, comme représenté sur la figure 8 annexée, sensiblement trois spires : une spire médiane de plus grand diamètre 110, 210, et deux spires latérales 120, 130 et 220, 230 respectivement. Pour garantir un effet de filtrage correct, les deux spirales doivent être espacées longitudinalement. Pour cela, les extrémités des deux spirales sont déformées dans des directions longitudinales respectivement opposées. Le cas échéant, les pas des spires latérales 120, 130, d'une part, 220, 230 d'autre part, des deux spirales peuvent être différents d'une spirale à l'autre pour éviter que les brins formés par ces spires latérales ne soient accolés même si les deux spires médianes 110, 210 sont portées en contact par l'élasticité de la veine cave. Le filtre de la figure 8 peut être formé de deux fils distinct dont les deux extrémités sont reliées par tout moyen classique approprié, tel que collage, brasure ou sertissage des embouts 300, 400. Ce filtre de la figure 8 peut être formé de deux fils distincts reliés entre eux à une seule de leurs extrémités, par tout moyen classique approprié, tel que collage, brasure ou sertissage d'un embout.

Le filtre de la figure 8 peut encore être réalisé avec un fil unique du type représenté sur l'une des figures 4, 5 ou 6.

Selon la figure 8 les deux spirales 100, 200 sont de même sens. En variante, les deux spirales 100, 200 pourraient être de sens opposés.

Selon encore une autre variante, le filtre peut être formé de deux spirales de diamètres constants et identiques pour les deux spirales.

Il peut s'agir de spirales de même sens ou de sens opposés.

Ces spirales peuvent être formées de deux fils distincts reliés respectivement à leurs deux extrémités, ou à une seule de leurs extrémités.

Ces spirales peuvent encore être formées d'un fil unique comme illustré sur les figures 4, 5 et 6.

Par ailleurs, le filtre conforme à la présente invention peut comprendre un nombre de spirales supérieures à deux, par exemple trois spirales, quatre spirales ou plus de diamètre constant ou non. Ces spirales peuvent être tous de même sens ou non. Elles peuvent être formées de fils distincts reliés respectivement à leurs deux extrémités. Les fils ont alors sensiblement tous la même longueur. Les spirales peuvent être formées de fils distincts reliés à l'une de leurs extrémités. Les spirales peuvent encore être formées d'un fil unique.

On a indiqué précédemment, que la première spirale composée du fil 100 comprend de préférence trois spires. Cette disposition s'est avérée optimum pour obtenir un effet de filtrage satisfaisant et un maintien correct du filtre sans risque de migration. Un plus grand nombre de spires accroîtrait inutilement la longueur du fil déroulé. Ceci aurait pour conséquence une difficulté accrue de transfert à travers le cathéter 800. En revanche, un nombre plus faible de spires réduirait l'effet de filtrage.

Toutefois, la définition de l'invention ne doit pas être limitée rigoureusement à une géométrie à trois spires. Il est clair en effet qu'un filtre ayant un nombre de spires légèrement supérieur ou inférieur à trois donnerait satisfaction.

### MISE EN PLACE DU FILTRE

La technique de mise en place du filtre conforme à la présente invention va maintenant être décrite en regard des figures 9 à 15, sur la base d'un filtre à deux spirales du type représenté sur les figures 1 à 3. Cette technique de mise en place s'applique cependant à l'une quelconque des variantes du filtre conforme à l'invention.

La qualité élastique des fils 100, 200 utilisés et leur faible diamètre permettent, sous l'effet d'une faible traction, de rendre le filtre provisoirement quasi-rectiligne. Dans cet état quasi-rectiligne le filtre peut être introduit à l'intérieur d'un cathéter 800 de petit diamètre, typiquement 7F comme indiqué précédemment pour y être véhiculé sans contrainte aussi bien dans un sens en vue d'une implantation dans la veine cave inférieure, que dans l'autre, en vue d'un retrait.

Le déplacement du filtre dans le cathéter 800 précité, en vue de la mise en place dans la veine cave peut être réalisé à l'aide de tous moyens classiques appropriés.

Le filtre peut être déplacé dans le cathéter 800 à l'aide d'un poussoir du type décrit dans les demandes de brevet antérieures FR-89 13160 du 9 Octobre 1989 et FR-89 16538 du 14 Décembre 1989. Le poussoir décrit dans ces demandes de brevet antérieures est illustré schématiquement sur les figures 9 et 10 annexées sous la référence 500.

Le poussoir 500 est fixé à l'extrémité d'un câble 550, tel qu'un câble d'acier souple tressé. Le câble 550 a typiquement un diamètre de l'ordre de 0,7mm. Ainsi, les tractions et poussées exercées sur le câble 550 permettent de manipuler le poussoir 500 et le filtre relié à celui-ci, dans le cathéter 800 de mise en place. Le poussoir 500 est formé d'un corps rigide d'enveloppe arrondie. Le poussoir 500 possède, sensiblement à mi-longueur, un évidemment 510 latéral conçu pour recevoir l'un des embouts 300, 400 prévu en extrémité du filtre, de préférence le cylindre d'extrémité 400. Par ailleurs, comme représenté sur la figure 10, le poussoir 500 possède, à l'opposé du câble 550, une encoche latérale 520 qui débouche dans l'évidemment 510. L'encoche 520 est conçue pour recevoir le ou les fils reliés à l'embout correspondant 300 ou 400.

On pourra se reporter à la description donnée dans les demandes de brevet FR-89 13160 du 9 Octobre 1989 et FR-89 16538 du 14 Décembre 1989 pour bien comprendre les opérations de mise en place du filtre à l'aide du poussoir 500 et du câble 550 associé.

On a indiqué précédemment que les deux fils 100, 200 peuvent être reliés à leurs extrémités par sertissage de l'embout 300 ou 400.

Comme représenté schématiquement sur les figures 9 et 10, en variante, on peut envisager de fixer l'une des extrémités, telles que l'extrémité distale 206, du deuxième fil 200 en retrait de l'extrémité correspondante 106 du premier fil 100, à l'aide d'une olive intermédiaire 450 ou de tout autre moyen classique approprié tel qu'un collage ou une brasure. De ce fait, comme représenté sur la figure 9, seul le premier fil 100 est engagé dans l'encoche 520 du poussoir. Cette disposition permet d'utiliser une encoche 520 de faible largeur.

Le poussoir 500 peut avoir typiquement une longueur de 10 à 12mm et un diamètre adapté au calibre interne du cathéter 800 de mise en place, typiquement de l'ordre de 1,6mm, pour pouvoir y glisser sans frottement. L'embout 400 échappe au poussoir 500 associé lorsque celui-ci émerge du cathéter de mise en place 800, grâce à l'élasticité du filtre, comme décrit dans les demandes de brevet antérieures 89 13160 du 9 Octobre 1989 et 89 16538 du 14 Décembre 1989.

On a représenté sur les figures 11 et 12 une autre variante de poussoir apte à véhiculer le filtre dans un cathéter de mise en place 800.

On aperçoit sur les figures 11 et 12 un poussoir 600 fixé sur un câble 650 similaire au câble 550 précité. Le poussoir 600 est muni d'une structure 610 du type fût fileté apte à être engagé dans un alésage taraudé correspondant 410 ménagé à l'extrémité de l'embout 400 ou 300. Le diamètre externe du poussoir 600 doit bien entendu être adapté pour permettre le coulissement du poussoir 600 dans le cathéter de mise en place 800. Pour assurer la mise en place d'un filtre à l'aide du poussoir 600 représenté sur les figures 11 et 12, on procède comme suit. Le filtre composé des fils 100, 200 est placé dans le cathéter de mise en place 800, son embout 400 étant relié au poussoir 600. Le cathéter 800 de mise en place est approché du lieu d'implantation du filtre dans la veine cave inférieure VC comme représenté sur la figure 13. Le positionnement du cathéter de mise en place 800 est contrôlé par des éléments radio-opaques prévus sur le cathéter 800, tels que par exemple deux bagues fines 810, 812 fixées à l'extrémité de celui-ci. (voir figure 16).

Par poussée sur le câble 650, le filtre est déplacé hors du cathéter de mise en place 800 dans la veine cave inférieure VC (voir figure 13). A la sortie du cathéter 800 le filtre reprend sa conformation en double spirale représentée sur les figures 1 et 2. Le maintien en place du filtre est assuré essentiellement par la spire médiane 110 de la première spirale qui s'appuie sur les parois de la veine cave VC, pour éviter tout risque de migration du filtre dans la veine cave VC. Les axes 102, 202 des spirales sont alors orthogonaux à l'axe longitudinal de la veine cave VC. Le diamètre de la spire médiane 110 étant de l'ordre de grandeur de la demi-circonférence de la veine cave inférieure, celle-ci est fortement aplatie après implantation du filtre comme représenté sur la figure 15. Le filtre induit de ce fait une forte diminution de la section de passage du flux sanguin dans la veine cave VC. Le filtrage des caillots venant de la circulation sanguine est du à deux effets cumulés : l'aplatissement de la veine cave dans la zone d'implantation comme indiqué précédemment d'une part, la présence des différents arcs composés par les deux spirales 100, 200 en travers de la veine cave d'autre part comme représenté sur la figure 15.

Sur ce point, la seconde spirale 200 qui présente un nombre de spires plus important que la première spirale en raison de son diamètre moyen plus faible joue un rôle prépondérant.

Pour séparer le filtre du poussoir 600 et du câble associé 650 une fois le filtre mis en place correctement, il suffit d'entraîner à rotation le câble 650 pour dévisser la structure 610 de l'alésage taraudé 410 complémentaire, comme illustré sur la figure 12.

Bien entendu, si le médecin constate une mauvaise mise en place du filtre, il peut retirer celui-ci à l'intérieur du cathéter de mise en place 800, tant que le poussoir 600 reste relié à l'embout 400.

En variante, on pourrait envisager de réaliser le fût fileté 610 sur l'embout 400 et l'alésage taraudé 410 dans le poussoir 600. Dans ce cas, ce fût 610 doit être conformé de telle sorte qu'il ne soit pas traumatique pour la veine cave.

### PRESENTATION DU FILTRE

Au stockage, le filtre peut être placé dans un tube conteneur approprié 700 comme représenté sur la figure 16. Le filtre conforme à la présente invention peut ainsi être vendu sous forme d'un kit comprenant le filtre composé des deux fils 100, 200, placé dans le tube conteneur précité 700, un cathéter de mise en place 800 conçu pour être relié au tube conteneur 700, et un poussoir 500 ou 600 relié à l'extrémité d'un câble 550 ou 650.

Toutefois le tube conteneur 700 n'est pas indispensable.

Le filtre anti-embolie pulmonaire conforme à la présente invention peut être conditionné sous blister. Il est alors introduit dans le cathéter de mise en place 800, par le médecin, juste avant l'implantation.

### MOYENS DE RETRAIT DU FILTRE

La conformation, non traumatisante pour la veine, du filtre, évite toute pénétration dans la paroi veineuse de quelques parties de ce filtre. Physiologiquement après implantation, les arcs des spires en contact avec la paroi veineuse sont recouverts progressivement de tissu endothélial. Le temps de fixation définitif du filtre dans la veine est au moins de l'ordre de quinze jours, de sorte que, pendant ce temps, il est toujours possible de procéder à son retrait percutané, c'est-à-dire sans opération chirurgicale.

Ce retrait peut être effectué comme décrit précédemment à l'aide des moyens évoqués dans les demandes de brevet FR-89 13160 du 9 Octobre 1989 et FR-89 16538 du 14 Décembre 1989.

Le retrait du filtre peut aussi être opéré à l'aide du dispositif de type "lasso" schématiquement représenté sur la figure 17.

De tels moyens de type "lasso" sont illustrés schématiquement sur la figure 17. Ils comprennent essentiellement un fil 900 muni d'une boucle 905 et engagée dans un cathéter 910.

Pour retirer le filtre d'une veine cave inférieure, il suffit d'approcher le cathéter 910 du lieu d'implantation, de saisir l'un des embouts 300, 400 et de retirer ainsi le filtre à l'intérieur du cathéter de retrait 910.

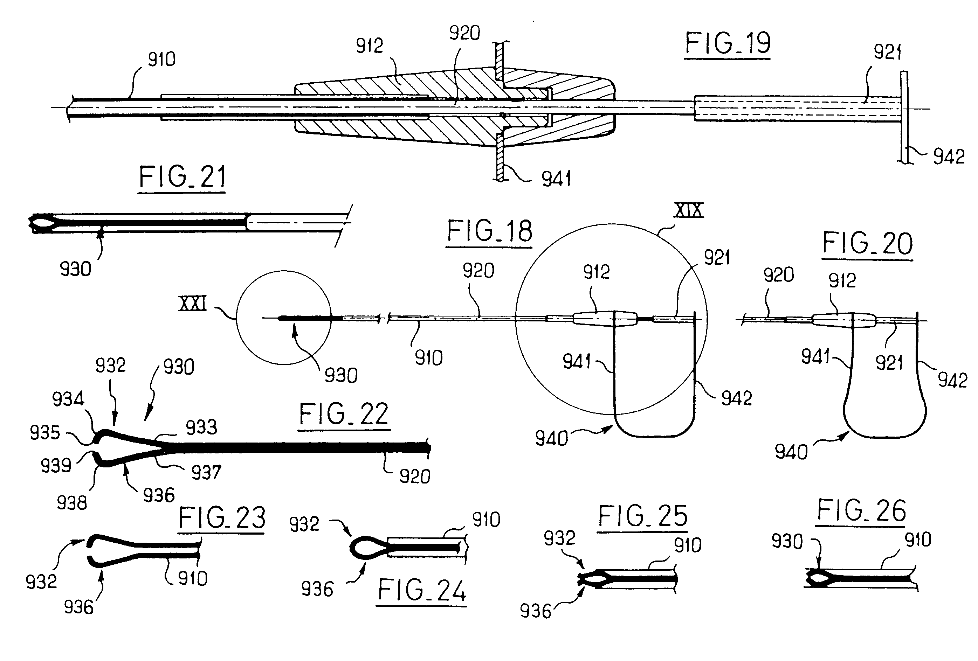

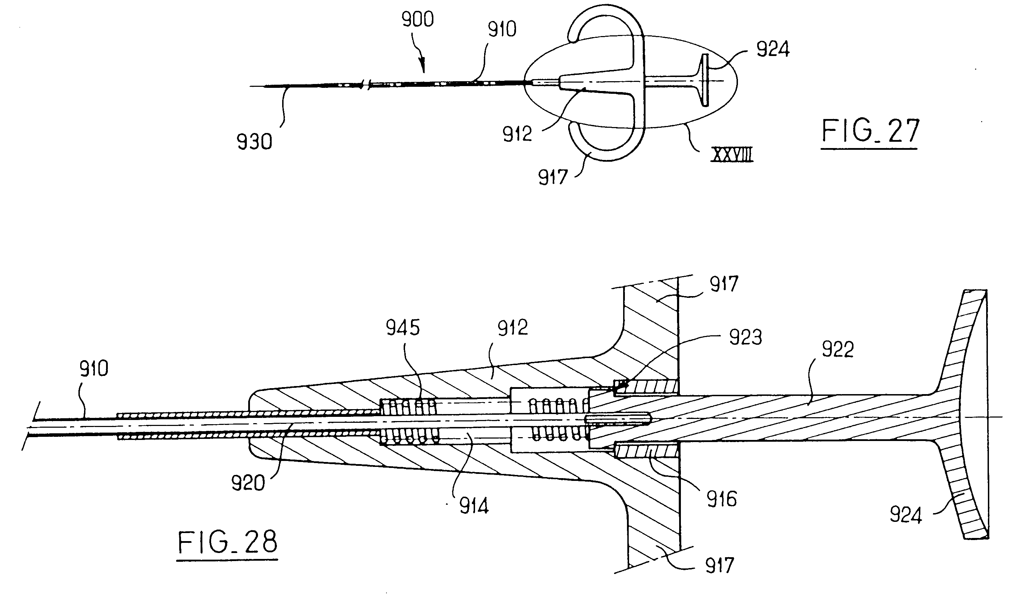

Le filtre peut encore être opéré à l'aide d'un instrument du type représenté sur les figures 18 à 26 ou du type représenté sur les figures 27 et 28.

On va maintenant décrire l'instrument représenté sur les figures 18 à 26.

Cet instrument 900 comprend essentiellement un tube 910 qui loge un poussoir allongé 920 muni d'une pince 930.

Le tube 910 et le poussoir 920 doivent être réalisés en matériau assez souple pour fléchir afin d'être introduit dans un cathéter lui-même placé dans un vaisseau conduisant au point d'implantation du filtre.

Le poussoir 920 peut coulisser dans le tube 910. La pince 930 est solidaire de l'extrémité proximale du poussoir 920. La pince 930 comprend deux mâchoires élastiques 932, 936.

Les deux mâchoires élastiques 932, 936 sont de préférence identiques et donc symétriques par rapport à l'axe du poussoir 920.

Les deux mâchoires 932, 936 sont conformées pour prendre au repos, en l'absence de contrainte externe, la configuration illustrée sur la figure 22.

C'est-à-dire qu'à partir de leur zone de raccordement sur le poussoir 920 les mâchoires 932, 936 divergent en 933, 937 en direction de leurs extrémités proximales, puis convergent en 934, 938.

Ainsi les deux extrémités 932, 936 présentent des concavités en regard.

Au repos les extrémités libres 935, 939 des deux mâchoires sont espacées pour permettre de placer un fil du filtre entre celles-ci.

Toutefois, le poussoir 920 et la pince 930 sont sollicités dans une position de retrait à l'intérieur du tube 910 par des moyens de rappel 940.

Les moyens de rappel 940 sont prévus à l'extrémité distale du tube 910 et du poussoir 920.

Ces moyens de rappel 940 peuvent faire l'objet de nombreux modes de réalisation.

En l'espèce, selon le mode de réalisation représenté sur la figure 18, ces moyens de rappel 940 comprennent une lame élastique en U, par exemple une lame métallique. Plus précisément, cette lame 940 est fixée, respectivement par ces deux branches parallèles 941, 942, sur un embout 912 solidaire de l'extrémité distale du tube 910 et l'extrémité distale 921 du poussoir 920.

Ainsi, comme représenté sur les figures 18, 21 et 26, au repos, les mâchoires 932, 936, de la pince 930 sont sollicitées, en position rétractée et serrée, dans le tube 910.

Pour ouvrir la pince 930, il suffit de solliciter en rapprochement les deux branches 941, 942, de la lame 940, comme représenté sur la figure 20. Ainsi le poussoir 920 est déplacé à translation dans le tube 910 et la pince 930 émerge alors à l'extrémité proximale de celui-ci. Les mâchoires 932, 936, reprennent ainsi leur position de repos illustrée sur les figures 22 et 23. Les deux mâchoires 932, 936, sont séparées. Dans cette position, un fil du filtre peut être engagé entre les mâchoires 932, 936. Pour saisir ce fil, il suffit de relacher la lame 940, tout en veillant à maintenir le fil entre les mâchoires 932, 936. Plus précisément, il faut pour cela, tout en maintenant la position du poussoir 920, laisser le tube se déplacer en direction proximale sous l'effet de la sollicitation de la lame 940.

Comme représenté sur la figure 24, le tube 910 prend alors appui sur les surfaces externes divergentes des mâchoires 932, 936, pour solliciter celles-ci à la fermeture.

Selon la souplesse du fil du filtre, le retrait des mâchoires 932, 936, dans le tube 910 peut être plus ou moins important.

La pince représentée sur les figures 18 à 26 peut être utilisée soit pour la mise en place du filtre, soit pour le retrait de celui-ci à travers un cathéter.

On comprendra aisément que la structure du poussoir 920 et de la pince 930 permet d'utiliser un tube 910, et donc un cathéter associé de faible diamètre.

On va maintenant décrire la variante de réalisation de l'instrument représenté sur les figures 27 et 28.

On retrouve sur ces figures un instrument 900 comprenant essentiellement un tube 910 qui loge un poussoir allongé 920 muni d'une pince 930.

L'instrument des figures 27 et 28 se distingue essentiellement de celui représenté sur les figures 18 à 20 par le fait que la lame en U est remplacée par un ressort spiral 945. Ce ressort 945 est placé dans un logement 914 formé dans l'embout 912 lié à l'extrémité distale du tube 910. Le ressort 945 est placé sur l'extrémité distale du poussoir 920, entre le fond du logement 914 et un embout 922 solidaire de l'extrémité distale du poussoir 920.

De préférence, il est prévu des moyens limitant le déplacement du poussoir 920 en direction distale sous l'effet du ressort 945. Ces moyens de limitation peuvent faire l'objet de nombreux modes de réalisation. Selon la figure 28, ces moyens de limitation comprennent un décrochement 923 formés sur l'embout 922 et prenant appui sur une bague 916 liée à l'embout 912.

L'extrémité proximale du poussoir 920 est muni d'une pince 930 conforme à celle précédemment décrite en regard des figures 21 à 26.

On notera par ailleurs à l'examen des figures 27 et 28, la présence de moyens de préhension 917, 924, respectivement sur les embouts 912 et 922, liés aux extrémités distales du tube 910 et du poussoir 920.

Ces moyens de préhension sont destinés à faciliter l'actionnement de l'instrument. Ils peuvent faire l'objet de nombreuses variantes. Pour cette raison, ces moyens de préhension ne seront pas décrits plus en détail par la suite.

Bien entendu la présente invention n'est pas limitée aux modes de réalisation particuliers qui viennent d'être décrits mais s'étend à toutes variantes conformes aux revendications suivantes.

Par exemple, bien que selon les modes de réalisation précédemment décrits et représentés sur les figures annexées les diverses spires soient sensiblement circulaires, on peut envisager d'utiliser des spires de configuration différentes, par exemple oblongues généralement ovales ou autres.

## Revendications

1. Filtre anti-embolie pulmonaire réalisé en un fil élastique à effet de ressort rémanent conformé en spiral caractérisé par le fait qu'il comprend :
- au moins une première spirale non plane (100), comportant sensiblement trois spires non jointives généralement circulaires :
. une spire médiane (110) dont le diamètre est sensiblement égal à la demi-circonférence de la veine cave dans la zone où le filtre doit être implanté, et
. deux spires latérales (120, 130) de plus faible diamètre que la spire médiane, et
- au moins une deuxième spirale non plane (200) non accolée à la première spirale, la première et la seconde spirale (100, 200) étant reliées au moins à l'une première de leurs extrémités.

2. Filtre anti-embolie pulmonaire selon la revendication 1, caractérisé par le fait qu'il est formé de plusieurs fils distincts (100, 200).

3. Filtre anti-embolie pulmonaire, selon la revendication 2, caractérisé par le fait que les fils distincts (100, 200) sont reliés respectivement à leurs deux extrémités.

4. Filtre anti-embolie pulmonaire selon la revendication 2, caractérisé par le fait que les fils distincts (100, 200) sont reliés à une seule de leurs extrémités.

5. Filtre anti-embolie pulmonaire selon l'une des revendications 3 ou 4, caractérisé par le fait que les fils (100, 200) sont reliés par des moyens choisis dans le groupe suivant : collage, brasure ou sertissage d'embouts (300, 400) rapportés.

6. Filtre anti-embolie pulmonaire selon la revendication 1, caractérisé par le fait qu'il est formé d'un fil unique.

7. Filtre anti-embolie pulmonaire selon la revendication 6, caractérisé par le fait que les deux extrémités du fil unique sont reliées entre elles par des moyens choisis dans le groupe suivant : collage, brasure, ou sertissage d'un embout.

8. Filtre anti-embolie pulmonaire selon la revendication 6, caractérisé par le fait que les extrémités libres du fil unique sont munies de boucles ouvertes (107, 207).

9. Filtre anti-embolie pulmonaire selon l'une des revendications 6 à 8, caractérisé par le fait que le fil unique est replié sur lui-même sensiblement à mi-longueur.

10. Filtre anti-embolie pulmonaire selon l'une des revendications 1 à 9, caractérisé par le fait que l'une au moins des extrémités du filtre est formée par un coude du fil.

11. Filtre anti-embolie pulmonaire selon l'une des revendications 1 à 10, caractérisé par le fait que l'une au moins des extrémités du filtre présente une discontinuité de courbure pour faciliter le retrait du filtre par saisie de cette extrémité.

12. Filtre anti-embolie pulmonaire selon l'une des revendications 1 à 11, caractérisé par le fait qu'il comprend des spirales (100, 200) de sens opposés.

13. Filtre anti-embolie pulmonaire selon l'une des revendications 1 à 12, caractérisé par le fait qu'il comprend deux spirales (100, 200) de même sens.

14. Filtre anti-embolie pulmonaire selon l'une des revendications 1 à 13, caractérisé par le fait que les diverses spirales (100, 200) ont au moins sensiblement la même longueur.

15. Filtre anti-embolie pulmonaire selon l'une des revendications 1 à 14, caractérisé par le fait que chaque fil élastique à effet de ressort rémanent (100, 200) est en métal.

16. Filtre anti-embolie pulmonaire selon l'une des revendications 1 à 15, caractérisé par le fait que chaque fil (100, 200) est réalisé à base d'un alliage choisi dans le groupe comprenant les alliages suivants :
i) cuivre et nickel et aluminium,
ii) cuivre et zinc et aluminium,
iii) cuivre et zinc et aluminium et nickel,
iv) cuivre et étain et nickel.

17. Filtre anti-embolie pulmonaire selon l'une des revendications 1 à 16, caractérisé par le fait que l'ensemble du filtre est revêtu d'une mince couche d'or.

18. Filtre anti-embolie pulmonaire selon l'une des revendications 1 à 17, caractérisé par le fait que la seconde spirale (200) comprend différentes spires de diamètres sensiblement identiques entre eux et inférieurs au diamètre de la spire médiane (110) de la première spirale.

19. Filtre anti-embolie pulmonaire selon l'une des revendications 1 à 18, caractérisé par le fait que la seconde spirale (200) comprend de l'ordre de quatre spires.

20. Filtre anti-embolie pulmonaire selon l'une des revendications 1 à 17, caractérisé par le fait que la seconde spire (200) à la même configuration que la première spirale : sensiblement trois spires non jointives dont une spire médiane (210) et deux spires latérales (220, 230) de plus faible diamètre que la spire médiane, les deux spirales étant décalées entre elles.

21. Filtre anti-embolie pulmonaire selon la revendication 20, caractérisé par le fait que le diamètre de la spire médiane (210) de la seconde spirale (200) est sensiblement égal à la demi-circonférence de la veine cave dans la zone où le filtre doit être implanté.

22. Filtre anti-embolie pulmonaire selon l'une des revendications 1 à 21, caractérisé par le fait que les diamètres des deux spires latérales (120, 130 ; 220, 230) sont sensiblement égaux entre eux.

23. Filtre anti-embolie pulmonaire selon l'une des revendications 1 à 22, caractérisé par le fait que les spires de la première spirale (100) sont coaxiales entre elles, et que les spires de la seconde spirale (200) sont coaxiales entre elles mais décentrées par rapport aux spires de la première spirale.

24. Filtre anti-embolie pulmonaire selon l'une des revendications 1 à 23, caractérisé par le fait que le diamètre des spires de la seconde spirale (200) est compris entre la moitié et le tiers du diamètre de la spire médiane de la première spirale (100).

25. Filtre anti-embolie pulmonaire selon l'une des revendications 1 à 17, caractérisé par le fait que les deux spirales (100, 200) comprennent plusieurs spires de diamètres constants et identiques, les deux spirales (100, 200) étant décalées entre elles.

26. Filtre anti-embolie pulmonaire selon l'une des revendications 1 à 25, caractérisé par le fait qu'elle comprend des fils (100, 200) de diamètres différents.

27. Filtre anti-embolie pulmonaire selon l'une des revendications 1 à 26, caractérisé par le fait que le premier fil (100) a un diamètre supérieur au second fil (200).

28. Filtre anti-embolie pulmonaire selon l'une des revendications 1 à 27, caractérisé par le fait que chaque fil (100, 200) a un diamètre compris entre 0,25mm et 1mm.

29. Filtre anti-embolie pulmonaire selon la revendication 27, caractérisé par le fait que le premier fil (100) a un diamètre de l'ordre de 0,37mm.

30. Filtre anti-embolie pulmonaire selon la revendication 29, caractérisé par le fait que le second fil (200) a un diamètre de l'ordre de 0,30mm.

31. Filtre anti-embolie pulmonaire selon l'une des revendications 1 à 30, caractérisé par le fait que la longueur des deux fils (100, 200) est comprise entre 22 et 35cm.

32. Filtre anti-embolie pulmonaire selon l'une des revendications 1 à 31, caractérisé par le fait que le diamètre de la spire médiane (110, 210) est supérieur ou égal à 1,25 fois le diamètre des spires latérales (120, 130 ; 220, 230).

33. Filtre anti-embolie pulmonaire selon l'une des revendications 1 à 32, caractérisé en ce que le diamètre (d110) de la spire médiane (110, 210) est supérieur ou égal à 1,4 fois le diamètre (d120, d130) des spires latérales (120, 130 ; 220, 230).

34. Filtre anti-embolie pulmonaire selon l'une des revendications 1 à 33, caractérisé en ce que les pas (p1, p2) des différentes spires de chaque spirale (100, 200) sont sensiblement égaux entre eux.

35. Filtre anti-embolie pulmonaire selon l'une des revendications 1 à 34, caractérisé en ce que le pas de la première spirale (100) au repos, avant implantation, est d'au moins 3mm.

36. Filtre anti-embolie pulmonaire selon l'une des revendications 1 à 35, caractérisé en ce que l'épaisseur axiale du filtre au repos, avant implantation, est d'au moins 9mm.

37. Filtre anti-embolie pulmonaire selon l'une des revendications 1 à 36, caractérisé en ce que le diamètre (d110) de la spire médiane est supérieur ou égal à deux fois, typiquement supérieur à 2,5 fois l'épaisseur axiale au repos, avant implantation du filtre.

38. Filtre anti-embolie pulmonaire selon l'une des revendications 1 à 37, caractérisé par le fait que les extrémités des fils (100, 200) sont munies d'embouts arrondis radio-opaques (300, 400).

39. Filtre anti-embolie pulmonaire selon la revendication 38, caractérisé par le fait que l'un au moins des embouts (300, 400) est muni d'une structure filetée et taraudée (610) apte à coopérer avec un poussoir complémentaire pour la mise en place du filtre.

40. Filtre anti-embolie pulmonaire selon l'une des revendications 1 à 39, caractérisé par le fait qu'il comprend un nombre de spirales supérieur à deux.

41. Kit de présentation et mise en place, caractérisé par le fait qu'il comprend :
- un filtre conforme à l'une des revendications 1 à 40,
- un cathéter (800) apte à recevoir le filtre à coulissement, et
- des moyens (600, 650) aptes à véhiculer le filtre dans le cathéter (800).

42. Kit selon la revendication 41, caractérisé par le fait que les moyens aptes à véhiculer le filtre comprennent un poussoir (600) muni d'une structure filetée ou taraudée (610) apte à coopérer avec un embout complémentaire (400) du filtre.

43. Kit selon l'une des revendications 41 ou 42, caractérisé par le fait qu'il comprend en outre un instrument de retrait comprenant un tube souple (910) qui loge un poussoir souple (920) muni d'une pince (930) à son extrémité proximale.

44. Kit selon la revendication 43, caractérisé par le fait que la pince (930) est formée de deux machoires élastiques symétriques (932, 936) sollicitées en écartement au repos.

45. Kit selon l'une des revendications 43 ou 44, caractérisé par le fait que des moyens élastiques (940, 945) sollicitent le poussoir et la pince (930) dans une position de repos rétractée dans le tube souple (910).

## Claims

1. Anti-pulmonary embolism filter, of the type made of a resilient wire with remanent spring effect shaped into a spiral, characterized in that it comprises:
- at least one first non-planar spiral (100), comprising substantially three generally circular non-touching turns:
. a middle turn (110) whose diameter is substantially equal to half the circumference of the vena cava in the zone where the filter is to be implanted, and
. two lateral turns (120, 130) of smaller diameter than the middle turn, and
- at least one second non-planar spiral (200), not attached to the first spiral, the first and the second spiral (100, 200) being connected by means of at least a first one of their ends.

2. Anti-pulmonary embolism filter according to Claim 1, characterized in that it is formed by a plurality of separate wires (100, 200).

3. Anti-pulmonary embolism filter according to Claim 2, characterized in that the separate wires (100, 200) are connected respectively at their two ends.

4. Anti-pulmonary embolism filter according to Claim 2, characterized in that the separate wires (100, 200) are connected at only one of their ends.

5. Anti-pulmonary embolism filter according to one of Claims 3 or 4, characterized in that the wires (100, 200) are connected by means chosen from the following group: adhesive bonding, soldering or crimping of attached endpieces (300, 400).

6. Anti-pulmonary embolism filter according to Claim 1, characterized in that it is formed by a single wire.

7. Anti-pulmonary embolism filter according to Claim 6, characterized in that the two ends of the single wire are connected together by means chosen from the following group: adhesive bonding, soldering or crimping of an endpiece.

8. Anti-pulmonary embolism filter according to Claim 6, characterized in that the free ends of the single wire are fitted with open loops (107, 207).

9. Anti-pulmonary embolism filter according to one of Claims 6 to 8, characterized in that the single wire is folded on itself substantially half-way along.

10. Anti-pulmonary embolism filter according to one of Claims 1 to 9, characterized in that at least one of the ends of the filter is formed by a bend in the wire.

11. Anti-pulmonary embolism filter according to one of Claims 1 to 10, characterized in that at least one of the ends of the filter has a discontinuity in curvature in order to facilitate withdrawal of the filter by grasping this end.

12. Anti-pulmonary embolism filter according to one of Claims 1 to 11, characterized in that it comprises spirals (100, 200) of opposite senses.

13. Anti-pulmonary embolism filter according to one of Claims 1 to 12, characterized in that it comprises two spirals (100, 200) of the same sense.

14. Anti-pulmonary embolism filter according to one of Claims 1 to 13, characterized in that the various spirals (100, 200) have at least substantially the same length.

15. Anti-pulmonary embolism filter according to one of Claims 1 to 14, characterized in that each elastic wire with remanent spring effect (100, 200) is made of metal.

16. Anti-pulmonary embolism filter according to one of Claims 1 to 15, characterized in that each wire (100, 200) is made based on an alloy chosen from the group comprising the following alloys:
i) copper and nickel and aluminium,
ii) copper and zinc and aluminium,
iii) copper and zinc and aluminium and nickel,
iv) copper and tin and nickel.

17. Anti-pulmonary embolism filter according to one of Claims 1 to 16, characterized in that the whole of the filter is coated with a thin layer of gold.

18. Anti-pulmonary embolism filter according to one of Claims 1 to 17, characterized in that the second spiral (200) comprises various turns whose diameters are substantially identical to each other and are less than the diameter of the middle turn (110) of the first spiral.

19. Anti-pulmonary embolism filter according to one of Claims 1 to 18, characterized in that the second spiral (200) comprises of the order of four turns.

20. Anti-pulmonary embolism filter according to one of Claims 1 to 17, characterized in that the second spiral (200) has the same configuration as the first spiral: substantially three non-touching turns, namely a middle turn (210) and two lateral turns (220, 230) of smaller diameter than the middle turn, the two spirals being mutually offset.

21. Anti-pulmonary embolism filter according to Claim 20, characterized in that the diameter of the middle turn (110) is substantially equal to half the circumference of the vena cava in the zone where the filter is to be implanted.

22. Anti-pulmonary embolism filter according to one of Claims 1 to 21, characterized in that the diameters of the two lateral turns (120, 130; 220, 230) are substantially equal to each other.

23. Anti-pulmonary embolism filter according to one of Claims 1 to 22, characterized in that the turns of the first spiral (100) are coaxial with each other, and in that the turns of the second spiral (200) are coaxial with each other but off-centred with respect to the turns of the first spiral.

24. Anti-pulmonary embolism filter according to one of Claims 1 to 23, characterized in that the diameter of the turns of the second spiral (200) lies between one half and one third of the diameter of the middle turn of the first spiral (100).

25. Anti-pulmonary embolism filter according to one of Claims 1 to 17, characterized in that the two spirals (100, 200) comprise a plurality of turns of constant and identical diameters, the two spirals (100, 200) being offset from each other.

26. Anti-pulmonary embolism filter according to one of Claims 1 to 25, characterized in that it comprises wires (100, 200) of different diameters.

27. Anti-pulmonary embolism filter according to one of Claims 1 to 26, characterized in that the first wire (100) has a diameter greater than the second wire (200).

28. Anti-pulmonary embolism filter according to one of Claims 1 to 27, characterized in that each wire (100, 200) has a diameter lying between 0.25 mm and 1 mm.

29. Anti-pulmonary embolism filter according to Claim 27, characterized in that the first wire (100) has a diameter of the order of 0.37 mm.

30. Anti-pulmonary embolism filter according to Claim 29, characterized in that the second wire (200) has a diameter of the order of 0.30 mm.

31. Anti-pulmonary embolism filter according to one of Claims 1 to 30, characterized in that the length of the two wires (100, 200) lies between 22 and 35 cm.

32. Anti-pulmonary embolism filter according to one of Claims 1 to 31, characterized in that the diameter of the middle turn (110, 210) is greater than or equal to 1.25 times the diameter of the lateral turns (120, 130; 220, 230).

33. Anti-pulmonary embolism filter according to one of Claims 1 to 32, characterized in that the diameter (d110) of the middle turn (110, 210) is greater than or equal to 1.4 times the diameter (d120, d130) of the lateral turns (120, 130; 220, 230).

34. Anti-pulmonary embolism filter according to one of Claims 1 to 33, characterized in that the pitches (p1, p2) of the various turns of each spiral (100, 200) are substantially equal to each other.

35. Anti-pulmonary embolism filter according to one of Claims 1 to 34, characterized in that the pitch of the first spiral (100) at rest, before implantation, is at least 3 mm.

36. Anti-pulmonary embolism filter according to one of Claims 1 to 35, characterized in that the axial thickness of the filter at rest, before implantation, is at least 9 mm.

37. Anti-pulmonary embolism filter according to one of Claims 1 to 36, characterized in that the diameter (d110) of the middle turn is greater than or equal to two times, typically greater than 2.5 times, the axial thickness at rest, before implantation, of the filter.

38. Anti-pulmonary embolism filter according to one of Claims 1 to 37, characterized in that the ends of the wires (100, 200) are fitted with rounded radiopaque endpieces (300, 400).

39. Anti-pulmonary embolism filter according to Claim 38, characterized in that at least one of the endpieces (300, 400) is fitted with a threaded or tapped structure (610) which can interact with a complementary pusher for fitting the filter.

40. Anti-pulmonary embolism filter according to one of Claims 1 to 39, characterized in that it comprises a number of spirals greater than two.

41. Presentation and fitting kit, characterized in that it comprises:
- a filter in accordance with one of Claims 1 to 40,
- a catheter (800) which can receive the filter in a sliding manner, and
- means (600, 650) capable of transporting the filter in the catheter (800).

42. Kit according to Claim 41, characterized in that the means capable of transporting the filter comprise a pusher (600) fitted with a threaded or tapped structure (610) capable of interacting with a complementary end-piece (400) of the filter.

43. Kit according to one of Claims 41 or 42, characterized in that it furthermore comprises a withdrawal instrument comprising a flexible tube (910) which accommodates a flexible pusher (920) fitted with a clamping means (930) at its proximal end.

44. Kit according to Claim 43, characterized in that the clamping means (930) is formed by two symmetrical resilient jaws (932, 936) pressed apart at rest.

45. Kit according to one of Claims 43 or 44, characterized in that the resilient means (940, 945) press the pusher and the clamping means (930) in a retracted rest position in the flexible tube (910).

## Patentansprüche

1. Anti-Lungenembolie-Filter, hergestellt aus einem elastischen Draht in Spiralform mit dauernder Federwirkung dadurch gekennzeichnet, daß es umfaßt:
- mindestens eine, nicht in einer Ebene liegende, erste Spirale (100), die genau drei, im allgemeinen kreisbogenförmige, einander nicht berührende Windungen aufweist;
- eine Mittelwindung (110), deren Durchmesser genau gleich dem halben Umfang der Hohlvene in dem Bereich ist, wo das Filter implantiert werden soll, und
- zwei Seitenwindungen (120, 130), deren Durchmesser kleiner ist als derjenige der Mittelwindung, sowie
- mindestens eine, nicht in einer Ebene liegende, nicht von der ersten Spirale umfaßte, zweite Spirale (200), wobei die erste und die zweite Spirale (100, 200) zumindest an einem ersten ihrer beiden Enden verbunden sind.

2. Anti-Lungenembolie-Filter nach Anspruch 1, dadurch gekennzeichnet, daß es aus mehreren verschiedenen Drähten (100, 200) besteht.

3. Anti-Lungenembolie-Filter nach Anspruch 2, dadurch gekennzeichnet, daß die verschiedenen Drähte (100, 200) jeweils an ihren beiden Enden verbunden sind.

4. Anti-Lungenembolie-Filter nach Anspruch 2, dadurch gekennzeichnet, daß die verschiedenen Drähte (100, 200) an einem ihrer Enden verbunden sind.

5. Anti-Lungenembolie-Filter nach einem der Ansprüche 3 oder 4, dadurch gekennzeichnet, daß die Drähte (100, 200) durch ein aus der folgenden Gruppe ausgewähltes Verfahren verbunden sind: Kleben, Löten oder Falzen aufgesteckter Hülsen (300, 400).

6. Anti-Lungenembolie-Filter nach Anspruch 1, dadurch gekennzeichnet, daß es aus einem einzigen Draht besteht.

7. Anti-Lungenembolie-Filter nach Anspruch 6, dadurch gekennzeichnet, daß die beiden Enden des einzigen Drahtes durch ein aus der folgenden Gruppe ausgewähltes Verfahren verbunden sind: Kleben, Löten oder Falzen aufgesteckter Hülsen.

8. Anti-Lungenembolie-Filter nach Anspruch 6, dadurch gekennzeichnet, daß die freien Enden des einzigen Drahtes mit offenen Ösen (107, 207) versehen sind.

9. Anti-Lungenembolie-Filter nach einem der Ansprüche 6 bis 8, dadurch gekennzeichnet, daß der einzige Draht genau in der Mitte seiner Länge umgebogen ist, so daß sich die beiden Enden in gleicher Richtung erstrecken.

10. Anti-Lungenembolie-Filter nach einem der Ansprüche 1 bis 9, dadurch gekennzeichnet, daß mindestens eines der Enden des Filters durch einen Drahtbogen gebildet wird.

11. Anti-Lungenembolie-Filter nach einem der Ansprüche 1 bis 10, dadurch gekennzeichnet, daß mindestens eines der Enden des Filters eine Unstetigkeit der Krümmung aufweist, um das Herausziehen des Filters durch Erfassen dieses Endes zu erleichtern.

12. Anti-Lungenembolie-Filter nach einem der Ansprüche 1 bis 11, dadurch gekennzeichnet, daß es Spiralen (100, 200) entgegengesetzter Wicklungsrichtung aufweist.

13. Anti-Lungenembolie-Filter nach einem der Ansprüche 1 bis 12, dadurch gekennzeichnet, daß es zwei Spiralen (100, 200) gleicher Wicklungsrichtung aufweist.

14. Anti-Lungenembolie-Filter nach einem der Ansprüche 1 bis 13, dadurch gekennzeichnet, daß die verschiedenen Spiralen (100, 200) zumindest annähernd die gleiche Länge haben.

15. Anti-Lungenembolie-Filter nach einem der Ansprüche 1 bis 14, dadurch gekennzeichnet, daß jeder der Drähte (100, 200) mit dauernder Federwirkung aus Metall besteht.

16. Anti-Lungenembolie-Filter nach einem der Ansprüche 1 bis 15, dadurch gekennzeichnet, daß jeder Draht (100, 200) aus einer Legierung hergestellt ist, die aus der Gruppe der folgenden Legierungen ausgewählt ist:
I) Kupfer, Nickel und Aluminium,
II) Kupfer, Zink und Aluminium,
III) Kupfer, Zink, Aluminium und Nickel,
IV) Kupfer, Zinn und Nickel.

17. Anti-Lungenembolie-Filter nach einem der Ansprüche 1 bis 16, dadurch gekennzeichnet, daß die Filter-Baugruppe mit einer dünnen Goldschicht überzogen ist.

18. Anti-Lungenembolie-Filter nach einem der Ansprüche 1 bis 17, dadurch gekennzeichnet, daß die zweite Spirale (200) mehrere Windungen mit untereinander genau gleichem Durchmesser aufweist, der zugleich kleiner ist als der Durchmesser der Mittelwindung (110) der ersten Spirale.

19. Anti-Lungenembolie-Filter nach einem der Ansprüche 1 bis 18, dadurch gekennzeichnet, daß die zweite Spirale (200) größenordnungsmäßig vier Windungen aufweist.

20. Anti-Lungenembolie-Filter nach einem der Ansprüche 1 bis 17, dadurch gekennzeichnet, daß die zweite Spirale (200) die gleiche Anordnung aufweist wie die erste Spirale, nämlich: genau drei einander nicht berührende Windungen, von denen eine die Mittelwindung (210) ist und die beiden anderen Seitenwindungen (220, 230) mit kleinerem Durchmesser als derjenige der Mittelwindung sind, wobei die beiden Spiralen gegeneinander verschoben sind.

21. Anti-Lungenembolie-Filter nach Anspruch 20, dadurch gekennzeichnet, daß der Durchmesser der Mittelwindung (210) der zweiten Spirale (200) genau gleich dem halben Umfang der Hohlvene in dem Bereich ist, wo das Filter implantiert werden soll.

22. Anti-Lungenembolie-Filter nach einem der Ansprüche 1 bis 21, dadurch gekennzeichnet, daß die Durchmesser der beiden Seitenwindungen (120, 130; 220; 230) untereinander genau gleich sind.

23. Anti-Lungenembolie-Filter nach einem der Ansprüche 1 bis 22, dadurch gekennzeichnet, daß die Windungen der ersten Spirale (100) untereinander koaxial sind und daß auch die Windungen der zweiten Spirale (200) untereinander koaxial aber gegenüber den Windungen der ersten Spirale dezentriert sind.

24. Anti-Lungenembolie-Filter nach einem der Ansprüche 1 bis 23, dadurch gekennzeichnet, daß der Durchmesser der Windungen der zweiten Spirale (200) zwischen der Hälfte und einem Drittel des Durchmessers der Mittelwindung der ersten Spirale (100) beträgt.

25. Anti-Lungenembolie-Filter nach einem der Ansprüche 1 bis 17, dadurch gekennzeichnet, daß die beiden Spiralen (100, 200) mehrere Windungen konstanten und identischen Durchmessers aufweisen und die beiden Spiralen (100, 200) gegeneinander verschoben sind.

26. Anti-Lungenembolie-Filter nach einem der Ansprüche 1 bis 25, dadurch gekennzeichnet, daß es Drähte unterschiedlichen Durchmessers aufweist.

27. Anti-Lungenembolie-Filter nach einem der Ansprüche 1 bis 26, dadurch gekennzeichnet, daß der erste Draht (100) einen größeren Durchmesser hat als der zweite Draht (200).

28. Anti-Lungenembolie-Filter nach einem der Ansprüche 1 bis 27, dadurch gekennzeichnet, daß jeder der Drähte (100, 200) einen Durchmesser zwischen 0,25 mm und 1 mm hat.

29. Anti-Lungenembolie-Filter nach Anspruch 27, dadurch gekennzeichnet, daß der erste Draht (100) einen Durchmesser in der Größenordnung von 0,37 mm hat.

30. Anti-Lungenembolie-Filter nach Anspruch 29, dadurch gekennzeichnet, daß der zweite Draht (200) einen Durchmesser in der Größenordnung von 0,30 mm hat.

31. Anti-Lungenembolie-Filter nach einem der Ansprüche 1 bis 30, dadurch gekennzeichnet, daß die Länge der beiden Drähte (100, 200) zwischen 22 und 35 cm liegt.

32. Anti-Lungenembolie-Filter nach einem der Ansprüche 1 bis 31, dadurch gekennzeichnet, daß der Durchmesser der Mittelwindung (110, 210) größer als das oder gleich dem 1,25fache(n) des Durchmessers der Seitenwindungen (120, 130, 220, 230) ist.

33. Anti-Lungenembolie-Filter nach einem der Ansprüche 1 bis 32, dadurch gekennzeichnet, daS der Durchmesser (d110) der Mittelwindung (110, 210) größer als das oder gleich dem 1,4fache(n) des Durchmessers (d120, d130) der Seitenwindungen (120, 130, 220, 230) ist.

34. Anti-Lungenembolie-Filter nach einem der Ansprüche 1 bis 33, dadurch gekennzeichnet, daß die Steigungen der verschiedenen Windungen jeder Spirale (100, 200) untereinander genau gleich sind.

35. Anti-Lungenembolie-Filter nach einem der Ansprüche 1 bis 34, dadurch gekennzeichnet, daß die Steigung der ersten Spirale (100) im Ruhezustand vor der Implantation mindestens 3 mm beträgt.

36. Anti-Lungenembolie-Filter nach einem der Ansprüche 1 bis 35, dadurch gekennzeichnet, daß die Axialabmessung des Filters im Ruhezustand vor der Implantation mindestens 9 mm beträgt.

37. Anti-Lungenembolie-Filter nach einem der Ansprüche 1 bis 36, dadurch gekennzeichnet, daß der Durchmesser (d110) der Mittelwindung größer als das oder gleich dem zweifache(n), typischerweise größer als das 2,5fache der Axialabmessung im Ruhezustand vor der Implantation ist.

38. Anti-Lungenembolie-Filter nach einem der Ansprüche 1 bis 37, dadurch gekennzeichnet, daß die Enden der Drähte (100, 200) mit abgerundeten, strahlungsundurchlässigen Hülsen (300, 400) versehen sind.

39. Anti-Lungenembolie-Filter nach einem der Ansprüche 1 bis 38, dadurch gekennzeichnet, daß mindestens eine der Hülsen (300, 400) mit einem Außen- oder Innengewinde (610) versehen ist, welches für den Eingriff eines komplemientären Stiftes zum Einsetzen des Filters eingerichtet ist.

40. Anti-Lungenembolie-Filter nach einem der Ansprüche 1 bis 39, dadurch gekennzeichnet, daß es mehr als zwei Spiralen aufweist.

41. Zusammenstellung zur Repräsentation und zum Einsetzen, dadurch gekennzeichnet, daß sie umfaßt:
- ein Filter nach einem der Ansprüche 1 bis 40,
- ein Katheter zur Aufnahme des Filters, das darin verschiebbar ist, und
- Einrichtungen (600, 650) zum Bewegen des Filters in dem Katheter (800).

42. Zusammenstellung nach Anspruch 41, dadurch gekennzeichnet, daß die Einrichtung zum Bewegen des Filters einen Stift mit einem Außen- oder Innengewinde (610) umfaßt, der zum Zusammenwirken mit einer komplementären Hülse (400) des Filters eingerichtet ist.

43. Zusammenstellung nach einem der Ansprüche 41 oder 42, dadurch gekennzeichnet, daß sie weiterhin ein Instrument zum Herausziehen enthält, welches ein biegsames Rohr (910) mit einem biegsamen Stift (920), der an seinem proximalen Ende mit einem Greifer (930) versehen ist, umfaßt.

44. Zusammenstellung nach Anspruch 43, dadurch gekennzeichnet, daß der Greifer (930) aus zwei symmetrischen elastischen Backen (932, 936) besteht, die im Ruhezustand in einem Abstand voneinander gehalten werden.

45. Zusammenstellung nach einem der Ansprüche 43 oder 44, dadurch gekennzeichnet, daß elastische Einrichtungen (940, 945) den Stift und den Greifer (930) in einer zusammengedrückten Ruhestellung in dem biegsamen Rohr (910) halten.
